# EUROPEAN PATENT SPECIFICATION

(11) **EP 2 759 272 B1**
(45) Date of publication and mention of the grant of the patent: **28.11.2018**
(21) Application number: 13152730.1
(22) Date of filing: 25.01.2013
(51) Int. Cl.: A61B 18/02

(54) **Device for non-surgical cold treatment of disorders**
Vorrichtung für nichtchirurgische Kaltbehandlung von Erkrankungen
Dispositif de traitement à froid non-chirurgical des troubles

(43) Date of publication of application: 30.07.2014
(73) Proprietor: YouMedical Brands B.V., 1059 AT Amsterdam (NL)
(72) Inventor: Witte, Johannes Hendricus, 1059 AT Amsterdam (NL); Stal, Robert Sebastiaan, 1059 AT Amsterdam (NL); Kneppers, Job, 2613 PZ Delft (NL)
(74) Representative: Brann AB

(56) References cited:
- EP-A1- 1 929 981
- GB-A- 2 079 608
- US-A- 4 345 598

## Description

The invention relates to a device for non-surgical cold treatment of disorders, comprising a container containing a refrigerant and having an outlet for the refrigerant and a valve communicating with the outlet, and an applicator mounted or mountable to the container, said applicator having an outer wall and forming a chamber at least when connected to the container.

Such devices are known from the prior art but most of them have not yet led to a successful use in practice. The devices that have reached commercial stage, which are mainly meant for the treatment of warts, are all open systems in which the refrigerant is supplied to the site to be treated. The refrigerant directly cools this site. The disadvantage of these devices is that the treatment is hardly controllable. The type of refrigerant determines the temperature and the cooling time is mainly determined by the refrigerant that is supplied to the site which is difficult to control. There is also a risk that the refrigerant damages or affects more skin than necessary for the treatment if refrigerant is spilled or supplied to the wrong place.

A device according to the preamble of claim 1 is disclosed in US 7,832,225. It shows a body surface cooling device having a dispensing channel connected to the outlet of the refrigerant container and being adapted to release refrigerant directly onto the inside face of covering parts of the cover of the device. The inside face of the covering parts thus operates as the heat exchanger and the outside face thereof as the contact member.

The present invention has as one of its objects to provide a novel device that is more practical and very efficient in use.

The device according to the invention has the features according to the characterizing portion of claim 1.

The porous heat exchanger can provide a very efficient exchange of cooling energy from the refrigerant to the contact member. Since the contact member is closed, refrigerant cannot arrive at the site to be treated, only cooling energy can reach this site. A control of the minimum temperature and the duration of the low temperature can be obtained if desired, for example by controlling the amount of refrigerant supplied to the heat exchanger or by the design of the heat exchanger. The shape of an outer surface of the contact member can easily be varied and adapted to the disorder to be treated, so that the device as a whole may be designed specifically for a wide range of disorders.

For low temperature cost effective heat exchangers (for example reaching temperatures of -10°C to -50°C), it is favourable to use a sintered metal for the porous member, for example sintered brass, although other metals having good heat conducting properties are conceivable, such as aluminium, copper, steel etc. For applications using higher temperatures, for example in the range from -10°C to +10°C it is also possible to make the heat exchanger partly or wholly from porous plastic.

To further improve the efficiency of the heat exchanger, the conductive porous member may at least partly be surrounded by a receptacle to receive any liquid refrigerant from the porous member therein, the receptacle being open to or vented to the chamber.

By receiving the refrigerant escaping from the porous member and keeping it close to the heat exchanger, the refrigerant will still cool the porous member even though it has left the porous member. If the receptacle and the porous member form a narrow space between them, the refrigerant will even be spread over the outer surface of the porous member to enhance transfer of cooling energy.

The receptacle may be directly attached to the applicator, in particular to the outer wall thereof, for example by a snap connection. This is a simple way of attaching the receptacle and possibly also the heat exchanger to the applicator.

In order to prevent liquid refrigerant to leave the applicator in case the applicator forms a chamber together with the container, the applicator connects to the container so as to hold fluid therein but to allow gas to escape. A slidable close contact between the applicator and the container may be sufficient for reaching this goal.

Such slidable contact is also useful if the applicator is at least slidable in axial direction with respect to the substantially cylindrical container in order to actuate the valve of the container.

In one embodiment, the applicator is provided with a child-proof lock to prevent unwanted actuation of the device.

In another embodiment the device is provided with a cover for the applicator covering the contact member. This especially useful if the device is actuated by depressing the applicator with respect to the container. This can then be done when the cover is on the applicator, avoiding the risk of touching the cold contact member during actuation.

The contact member protruding through the outer wall of the applicator and the heat exchanger fixed thereto may be sealed with respect to the outer wall at an inner surface thereof. Due to the temperature differences in the device (and the resulting dimensional changes in the heat exchanger), it is easier to obtain a seal in axial direction than in radial direction, for example by allowing the heat exchanger to be in pressing contact with the inner surface of the outer wall of the applicator.

The applicator can be made removable from the container, the applicator having a substantially cylindrical sleeve portion which is slidably guided on the outer surface of the container, but allowing the applicator to slide off of the container. If the applicator is not removable, the sleeve can be shorter, but there will be a stop preventing removal.

In one embodiment, the substantially cylindrical contact member slightly protrudes through the outer wall of the applicator and terminates in a substantially circular shaped skin contact surface, and the outer wall of the applicator may substantially have the shape of a dome extending concentrically around the contact member. Also disclosed is a method of operating a device for the cold treatment of disorders. It comprises the steps of providing a device having a container containing a refrigerant and including an outlet with a valve actuated by pressing, an applicator mounted on the container and connected to the outlet, a contact member mounted in and protruding from the applicator, and preferably a cover removably mounted on the applicator, depressing the cover or applicator to actuate the valve through the applicator, waiting until the temperature of the contact member has reached the right temperature and removing the cover from the applicator, if applicable, applying the contact member of the applicator, wherein the cover and/or applicator is first rotated with respect to the container from a position in which depression of the applicator is prevented to a position in which depression of the applicator is allowed before depressing the cover or applicator.

Further details and advantages of the invention will follow from the following description with reference to the accompanying drawings showing exemplary embodiments of the invention.
Figs. 1 - 4 are perspective views, partially broken away, showing 4 different embodiments of the device of the invention.
Figs. 5 - 8 are side views of the devices shown in Figs. 1 - 4.
Figs. 9 - 12 are sectional views according to the lines IX-IX, X-X, XI-XI and XII-XII in Figs. 5 - 8.

The drawings show several embodiments of a device for the cold treatment of human body disorders, in particular skin disorders like warts, age spots, skin tags, bruises or swellings, but also disorders in external body cavities like nose, ear, mouth and also for example piles in the anus. These disorders can be treated non-surgically by supplying cooling energy to the site, i.e. epicutaneous. This can be done by a physician or by the patient him/herself. In some applications, for example with warts, the temperature of the device should be very low, in the order of -30°C to -50°C, in order to freeze the disorder. In other applications, for example with swellings, the temperature can be much higher, around 0°C.

The devices shown in the drawings are all intended for treating warts with deep freezing temperatures.

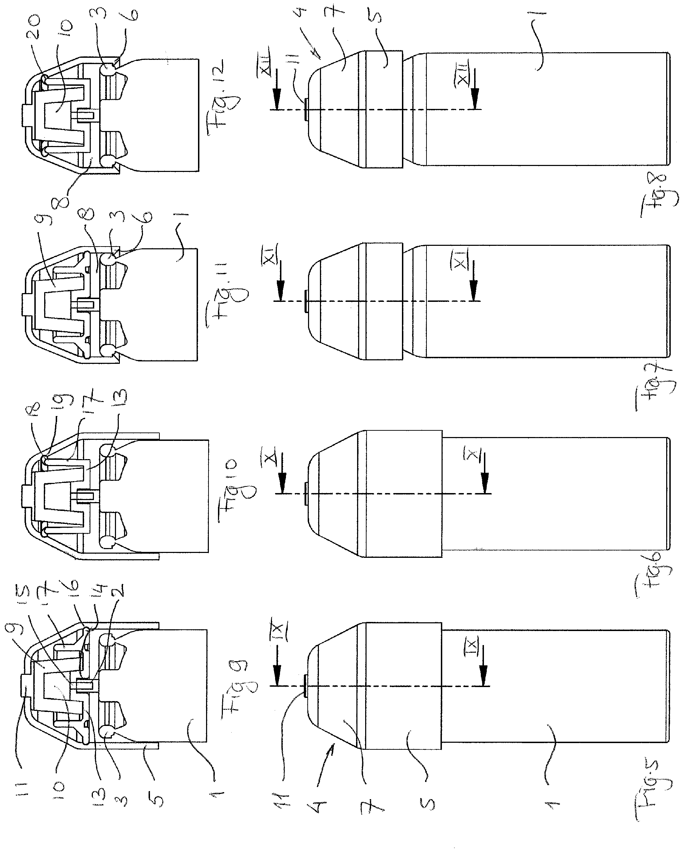

The device includes a container 1 containing a refrigerant. The refrigerant can be selected depending on the particular application of the device and the required temperatures. In this case it is an aerosol containing butane/propane, but can also be liquefied CO₂ or any other useful refrigerant. Refrigerant is to mean any liquid or gas which is able to extract heat due to evaporation and/or expansion or other cooling processes. So, for example, also propellants that have a cooling effect is considered to be a refrigerant. The container may be a standard spray or aerosol can having a valve (not shown) and an outlet tube 2 (see Figs. 9 - 12). The can may have a capacity of 25 - 250 ml, more particular 50 - 100 ml, for example. The container 1 is more or less cylindrical having a collar 3 spaced around the outlet tube 2.

An applicator 4 is mounted on container 1 at the end containing outlet tube 2. For this mounting purpose, applicator 4 comprises a sleeve portion 5 adapted to engage around the outer surface of container 1.

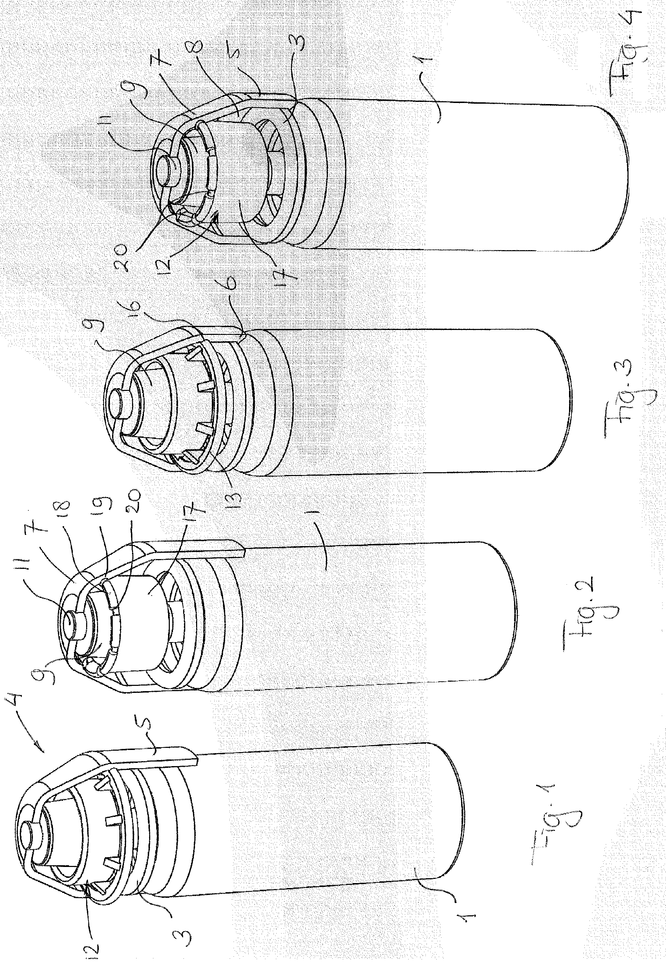

In the embodiments of Fig. 1 and 2, sleeve portion 5 is relatively long and engages around a considerable length of container 1, at least up to the cylindrical main portion of the container. In this embodiment, applicator 4 is removable from container 1. The sleeve portion 5 is just engaging around container 1 with a sliding (frictional) fit and without any stop preventing removal of applicator 4.

In the embodiment of Figs. 3 and 4, sleeve portion 5 is shorter, but comprises an inner ridge 6 or the like at the open end of sleeve portion 5 engaging behind collar 3 of container 1, thus functioning as a stop preventing removal of applicator 4 and determining the non-actuating position of the applicator on the outlet tube 2 and valve.

In both embodiments, the valve of container 1 is actuated by depressing outlet tube 2 by means of applicator 4 which must thus also be depressed. During this sliding movement, the applicator is guided by sleeve portion 2 engaging around container 1 and by outlet tube 2.

The sleeve portion 5 of applicator 4 is part of an outer wall thereof and connects to a dome-shaped wall portion 7. These wall portions 5, 7 together with the top of container 1 define a chamber 8 within applicator 4. The sleeve portion 5 may be sealed to container 1 in such a way that it prevents leakage of liquid refrigerant from chamber 8, but allows the passage of gaseous refrigerant.

Mounted within chamber 8 is a heat exchanger comprising a porous member 9, for example made from a material having good heat conducting properties. One example thereof is sintered metal such as brass having pores allowing passage of refrigerant. The porous member 9 is positioned around the outlet opening of outlet tube 2 of container 1. The porous member 9 has a cavity 10 (see Figs. 9 - 12) where the refrigerant from outlet tube 2 enters and where it will expand thereby absorbing energy from the cavity and surrounding porous member 9 thus effecting a cooling thereof. On the end of porous member 9 remote from outlet tube 2 and adjacent the wall portion of applicator 4, there is provided a contact member 11 to contact the site to be treated. This contact member and/or the heat exchanger is closed such that refrigerant cannot escape from the heat exchanger or chamber 8 through this contact member 11. This contact member 11 can be a separate part attached to porous member 9, but it may also be integrated in the porous member, while it is made impermeable, for example by melting, impregnating or adding a separate part or layer of metal or plastic to porous member 9. The contact member 11 is mainly cylindrical and slightly protrudes through an opening in the top of the dome shaped wall portion 7 of applicator 4, which is flattened in the embodiment shown, so that wall portion 9 is substantially in the form of a truncated cone, having in its centre the circular outer surface of contact member 11. However, contact member 11 may be covered by a thin protective layer of for example plastic in some embodiments. The contact member does not have to protrude completely through the outer wall of applicator 4. It just needs to be exposed to such an extent that it can cool the site to be treated to a desired extent.

The porous member 9 is mounted within applicator 4 by means of a mounting member 12. This mounting member 12 is here formed as a separate plastic part. It has a bottom portion 13 including in its centre a cavity 14 in which outlet tube 2 fits. Cavity 14 communicates with a passage 15 through bottom portion 13 to allow refrigerant to enter cavity 10 within porous member 9 through bottom portion 13 of mounting member 12.

In the embodiments of Figs. 1 and 3, bottom portion 13 extends up to the outer wall of applicator 4, in particular sleeve portion 5 thereof. This is provided with a circumferential inner groove 16 in which the circumferential edge of bottom portion 13 can snap to attach the mounting member 12 to the outer wall of applicator 4 and thereby also mounting porous member 9 and contact member 11. In fact, when mounting member 12 is seated with its edge in groove 16 it presses porous member 9 against the outer wall of applicator 4 surrounding the opening for contact member 11. This pressing contact enables a gas tight seal (with or without any assistance of a sealant) between the upper surface of porous member 9 and the inner surface of outer wall portion 7 of applicator 4 thus preventing escape of refrigerant there, also with varying temperatures of porous member 9 due to the refrigerant.

The mounting member 12 in the embodiments of Figs. 1 and 3 includes an upright wall member 17 forming together with bottom portion 13 a receptacle for any liquid refrigerant escaping from porous member 9. The wall member ends at a distance from the outer wall of applicator 4, so that receptacle is open to chamber 8 of the applicator. The wall member 17 closely surrounds the outer surface of porous member 9 thus only leaving a narrow annular space between them thereby forcing any liquid refrigerant to stay in contact with the outer surface of porous member 9 and thus withdrawing heat from porous member 9. This improves the efficiency of the heat exchanger.

When during normal use of the device the valve of the container is actuated with the device in an upright position, all liquid refrigerant will be received in the receptacle and will be evaporated before the applicator is moved to an upside down position in which contact member 11 is exposed to the site to be treated. The bottom portion 13 outside upright wall member 17 will normally be open to allow gaseous refrigerant to escape.

In the embodiments of Figs. 2 and 4, mounting member 12 is formed differently. Bottom portion 13 is now terminated short of sleeve portion 5, but upright wall member 17 is extended up to inner surface of dome shaped wall portion 7 and the upper end of wall member 17 is provided with a collar 18 adapted to snap into a groove 19 in the inner surface of outer wall portion 7. In order to vent the receptacle to chamber 8 of the applicator, one or more recesses 20 or openings are provided in collar 18 (see Figs. 2 and 4) so that gaseous refrigerant can escape from the receptacle to chamber 8 and then to the environment.

Not shown in the drawings is a cover that fits onto the applicator to cover the contact member. The cover or the applicator may be provided with a child lock, for example such that the cover and applicator can only be depressed after a small rotation of the cover and/or applicator. This can be effected by providing a member allowing depression of the applicator in one rotational position and preventing depression in another rotational position.

Use of the devices as shown is as follows. In the unlocked position, applicator 4 is depressed with respect to container 1 against spring pressure of the valve, preferably with the cover on the applicator. Depressing is continued until a sufficient amount of refrigerant from container 1 has entered cavity 10 within porous member 9 of the heat exchanger. There the liquid refrigerant will evaporate and withdraw heat from the porous member so that the temperature thereof will drop. The pores of the porous member 9 will enable a good and wide spread contact between the refrigerant and the material of porous member 9. Due to the good heat conducting properties, also the temperature of contact member 11 will drop to the same degree, depending on the type of and amount of refrigerant supplied to the heat exchanger. Any liquid refrigerant that passes through the porous member will be received in the receptacle formed by bottom portion 13 and upright wall member 17 and thus is kept in close contact with the heat exchanger. If the pressure in chamber 8 is rising above that of the environment, gaseous refrigerant may escape between the sleeve portion 5 and outer surface of container 1. Due to the pressure between the upper surface of porous member 9 and the lower surface of outer wall portion 7 exerted by mounting member 12 and by the user depressing the applicator, no gaseous refrigerant will escape at the position of contact member 11.

After a prescribed time, the temperature of contact member 11 will be at the required level and the user may stop depressing applicator 4 so that it will return to its rest position by the spring force of the valve of container 1. The user may remove the cover, if he/she has not already done so. The contact member 11 may then be brought into contact with the site to be treated, for example with the wart. The wart will be frozen by the contact member and due to the limited surface that is at such lowered temperature, there is no risk of surroundings of the wart being frozen, so that there is hardly any risk of skin burns. In this application, the upper surface of the contact member may for example be between 3 and 5 mm, in particular substantially 4 mm.

In the embodiment of Figs. 1 and 2, the applicator may or may not be removed from the container before it is applied to the site to be treated.

From the foregoing it will be clear that the invention provides a device which is very easy to handle and operate without the risk of skin burns. The device can be easily controlled either by design and/or operation. For example, temperature can be controlled either by the choice of material for the heat exchanger/contact member or by regulating the amount of refrigerant supplied. This can be done either manually or automatically by for example a time switch or bimetal switch in the supply.

The invention is not limited to the embodiments shown in the drawings and described above which can be varied in different manners within the scope of the invention. First of all, it is noted that features of different embodiments can be used in other combinations. Furthermore, it is possible to replace parts of the device by alternative arrangements. For example, in stead of a sintered porous member, it is possible to use a member having one or more channels or passages formed therein allowing close contact between refrigerant and heat exchanger. The receptacle to keep escaping liquid refrigerant in close contact with the heat exchanger can be combined with different types of heat exchangers. The mounting member and the wall portions of the applicator are generally made of plastic material. Especially the outer wall of the applicator is preferably made of material having a low heat conductivity (much lower than that of the porous member and contact member, so that the temperature of the applicator does not lower too much. At least part of the mounting member or receptacle may also be made in one piece with the heat exchanger. In other applications, especially those with moderately reduced temperatures, such as for treating swellings, the outer surface of the contact member will be much larger to contact a larger surface of the skin. Thus, the shape of the outer surface of the contact member will be varied in accordance with the disorder to be treated. In case of an applicator for treating piles in the anus, the applicator will have a narrower elongated part containing the contact member so that it can be inserted in the anus and the contact member surface will (also) be on the circumference of the elongated part, not (only) at the end thereof. The contact member surface may be covered by a thin layer of plastic. Generally, the applicator and the container will be a disposable unit. However, especially with the embodiment in which the applicator is removable from the container, it is possible to have a reusable applicator sold separately, so that if the container is empty it can be replaced by a new one to which the old applicator is mounted. Therefore an example not according to the invention also relates to an applicator without the container, which is adapted to be mounted to a fitting container. The valve for supplying refrigerant may, not according to the invention, also be present in the applicator.

## Claims

1. Device for non-surgical cold treatment of disorders, comprising:
a container (1) containing a refrigerant and having an outlet (2) for the refrigerant and a valve communicating with the outlet,
an applicator (4) mounted or mountable to the container, said applicator having an outer wall (7) and forming a chamber (8) at least when connected to the container,
a heat exchanger within the chamber (8) in fluid communication with the outlet (2) of the container (1) and receiving refrigerant when the valve of the container is opened,
a closed contact member (11) in heat exchange contact with the heat exchanger and being exposed to an outer side of the applicator,
**characterized in that** the heat exchanger comprises a porous member (9) made of a material having a high thermal conductivity and adapted to conduct fluid refrigerant from the outlet (2) of the container (1) to the chamber (8) while the refrigerant extracts heat from the heat exchanger and the contact member (11).

2. Device of claim 1, wherein the porous member (9) is made of a sintered material, such as metal like brass, or plastic.

3. Device of claim 1 or 2, wherein the conductive porous member (9) is at least partly surrounded by a receptacle (13, 17) to receive any liquid refrigerant from the porous member therein, the receptacle being open to or vented to the chamber (8).

4. Device of claim 3, wherein the receptacle (13, 17) and the porous member (9) form a narrow space between them.

5. Device of claim 3 or 4, wherein the receptacle (13, 17) is directly attached to the applicator (4), in particular to the outer wall (7) thereof, for example by a snap connection (18, 19).

6. Device of any of the preceding claims, wherein the applicator (4) connects to the container (1) so as to hold fluid therein but to allow gas to escape.

7. Device of any of the preceding claims, wherein the applicator (4) is at least slidable in axial direction with respect to the substantially cylindrical container (1) in order to actuate the valve of the container, and wherein the applicator is optionally provided with a child-proof lock.

8. Device of any of the preceding claims, provided with a cover for the applicator (4) covering the contact member (11).

9. Device of any of the preceding claims, wherein the contact member (11) protruding through the outer wall (7) of the applicator (4) and the heat exchanger fixed thereto are sealed with respect to the outer wall at an inner surface thereof, and wherein the heat exchanger is optionally in pressing contact with the inner surface of the outer wall of the applicator.

10. Device of any of the preceding claims, wherein the applicator (4) is removable from the container (1), the applicator having a substantially cylindrical sleeve portion (5) which is slidably guided on the outer surface of the container.

11. Device of any of the preceding claims, wherein the substantially cylindrical contact member (11) slightly protrudes through the outer wall (7) of the applicator (4) and preferably terminates in a substantially circular contact surface.

12. Device of claim 11, wherein the outer wall (7) of the applicator (4) has substantially the shape of a dome concentrically around the contact member (11).

## Patentansprüche

1. Vorrichtung zur nicht-chirurgischen Kältebehandlung von Erkrankungen, umfassend:
einen Behälter (1), der ein Kältemittel enthält und einen Ausgang (2) für das Kältemittel und ein Ventil aufweist, das mit dem Ausgang verbunden ist,
einen Applikator (4), der am Behälter montiert oder montierbar ist, wobei der Applikator eine Außenwand (7) aufweist und mindestens bei Verbindung mit dem Behälter eine Kammer (8) bildet,
einen Wärmetauscher innerhalb der Kammer (8) in Fluidverbindung mit dem Ausgang (2) des Behälters (1) und der Aufnahme von Kältemittel, wenn das Ventil des Behälters geöffnet wird,
ein geschlossenes Kontaktelement (11) in Wärmeaustauschkontakt mit dem Wärmetauscher und das einer Außenseite des Applikators ausgesetzt ist,
**dadurch gekennzeichnet, dass** der Wärmetauscher ein poröses Element (9) umfasst, das aus einem Material mit hoher Wärmeleitfähigkeit hergestellt ist und dazu ausgelegt ist, flüssiges Kältemittel vom Ausgang (2) des Behälters (1) zur Kammer (8) zu leiten, während das Kältemittel Wärme aus dem Wärmetauscher und dem Kontaktelement (11) entzieht.

2. Vorrichtung nach Anspruch 1, wobei das poröse Element (9) aus einem gesinterten Material, wie Metall, wie Messing, oder Kunststoff, hergestellt ist.

3. Vorrichtung nach Anspruch 1 oder 2, wobei das leitende poröse Element (9) mindestens teilweise von einem Gefäß (13, 17) umgeben ist, um flüssiges Kältemittel von dem porösen Element darin aufzunehmen, wobei das Gefäß zur Kammer (8) hin offen oder belüftet ist.

4. Vorrichtung nach Anspruch 3, wobei das Gefäß (13, 17) und das poröse Element (9) einen engen Raum zwischen ihnen bilden.

5. Vorrichtung nach Anspruch 3 oder 4, wobei das Gefäß (13, 17) direkt am Applikator (4), insbesondere an dessen Außenwand (7), zum Beispiel durch eine Schnappverbindung (18, 19), befestigt ist.

6. Vorrichtung nach einem der vorstehenden Ansprüche, wobei der Applikator (4) mit dem Behälter (1) verbunden ist, um Fluid darin zu halten, aber das Gas entweichen zu lassen.

7. Vorrichtung nach einem der vorstehenden Ansprüche, wobei der Applikator (4) mindestens in axialer Richtung in Bezug auf den im Wesentlichen zylindrischen Behälter (1) verschiebbar ist, um das Ventil des Behälters zu betätigen, und wobei der Applikator gegebenenfalls mit einer Kindersicherung versehen ist.

8. Vorrichtung nach einem der vorstehenden Ansprüche, versehen mit einer Abdeckung für den Applikator (4), der das Kontaktelement (11) bedeckt.

9. Vorrichtung nach einem der vorstehenden Ansprüche, wobei das Kontaktelement (11), das durch die Außenwand (7) des Applikators (4) und den daran befestigten Wärmetauscher ragt, in Bezug auf die Außenwand an einer Innenfläche davon abgedichtet ist, und wobei der Wärmetauscher gegebenenfalls in Druckkontakt mit der Innenfläche der Außenwand des Applikators steht.

10. Vorrichtung nach einem der vorstehenden Ansprüche, wobei der Applikator (4) aus dem Behälter (1) herausnehmbar ist, wobei der Applikator einen im Wesentlichen zylindrischen Hülsenabschnitt (5) aufweist, der auf der Außenfläche des Behälters verschiebbar geführt ist.

11. Vorrichtung nach einem der vorstehenden Ansprüche, wobei das im Wesentlichen zylindrische Kontaktelement (11) etwas durch die Außenwand (7) des Applikators (4) ragt und vorzugsweise in einer im Wesentlichen kreisförmigen Kontaktfläche endet.

12. Vorrichtung nach Anspruch 11, wobei die Außenwand (7) des Applikators (4) im Wesentlichen die Form einer Kuppel aufweist, die konzentrisch um das Kontaktelement (11) angeordnet ist.

## Revendications

1. Dispositif de traitement à froid non chirurgical de désordres, comprenant :
un récipient (1) contenant un réfrigérant et comportant une sortie (2) pour le réfrigérant et une valve communiquant avec la sortie ;
un applicateur (4) monté ou pouvant être monté sur le récipient, ledit applicateur comportant une paroi externe (7) et formant une chambre (8) au moins lorsqu'il est relié au récipient ;
un échangeur de chaleur à l'intérieur de la chambre (8) en communication de fluide avec la sortie (2) du récipient (1) et recevant du réfrigérant lorsque la valve du conteneur est ouverte ;
un élément de contact fermé (11) en contact d'échange de chaleur avec l'échangeur de chaleur et qui est exposé à un côté externe de l'applicateur ;
**caractérisé en ce que** l'échangeur de chaleur comprend un élément poreux (9) constitué d'un matériau présentant une conductivité thermique élevée et adapté à conduire du réfrigérant fluide depuis la sortie (2) du récipient (1) vers la chambre (8) tandis que le réfrigérant extrait de la chaleur de l'échangeur de chaleur et de l'élément de contact (11) .

2. Dispositif selon la revendication 1, dans lequel l'élément poreux (9) est constitué d'un matériau fritté, tel qu'un métal comme le laiton, ou du plastique.

3. Dispositif selon la revendication 1 ou 2, dans lequel l'élément poreux conducteur (9) est au moins partiellement entouré par un réceptacle (13, 17) destiné à y recevoir tout réfrigérant liquide provenant de l'élément poreux, le réceptacle étant ouvert sur la chambre (8) ou évacué vers celle-ci.

4. Dispositif selon la revendication 3, dans lequel le réceptacle (13, 17) et l'élément poreux (9) forment un espace étroit entre eux.

5. Dispositif selon la revendication 3 ou 4, dans lequel le réceptacle (13, 17) est directement attaché à l'applicateur (4), en particulier au niveau de sa paroi extérieure (7), par exemple par une liaison par encliquetage (18, 19).

6. Dispositif selon l'une quelconque des revendications précédentes, dans lequel l'applicateur (4) est relié au récipient (1) de manière à y contenir du fluide mais à permettre au gaz de s'en échapper.

7. Dispositif selon l'une quelconque des revendications précédentes, dans lequel l'applicateur (4) est au moins coulissant dans la direction axiale par rapport au récipient sensiblement cylindrique (1) afin d'actionner la valve du récipient, et dans lequel l'applicateur est optionnellement muni d'une serrure de sécurité pour enfant.

8. Dispositif selon l'une quelconque des revendications précédentes, muni d'un couvercle pour l'applicateur (4) recouvrant l'élément de contact (11).

9. Dispositif selon l'une quelconque des revendications précédentes, dans lequel l'élément de contact (11) faisant saillie à travers la paroi externe (7) de l'applicateur (4) et l'échangeur de chaleur fixé à celle-ci sont scellés par rapport à la paroi externe sur une surface interne de celle-ci, et dans lequel l'échangeur de chaleur est optionnellement en contact par pression avec la surface interne de la paroi externe de l'applicateur.

10. Dispositif selon l'une quelconque des revendications précédentes, dans lequel l'applicateur (4) peut être retiré du récipient (1), l'applicateur comportant une partie manchon sensiblement cylindrique (5) qui est guidée de manière coulissante sur la surface extérieure du récipient.

11. Dispositif selon l'une quelconque des revendications précédentes, dans lequel l'élément de contact (11) sensiblement cylindrique fait légèrement saillie à travers la paroi externe (7) de l'applicateur (4) et se termine de préférence dans une surface de contact sensiblement circulaire.

12. Dispositif selon la revendication 11, dans lequel la paroi externe (7) de l'applicateur (4) a sensiblement la forme d'un dôme concentrique autour de l'élément de contact (11).
